# EUROPEAN PATENT SPECIFICATION

(11) **EP 1 928 913 B1**
(45) Date of publication and mention of the grant of the patent: **26.06.2013**
(21) Application number: 06777156.8
(22) Date of filing: 04.09.2006
(51) Int. Cl.: C07K 16/18, A61K 39/395, A61K 39/00, A61P 9/10, A61K 31/33, G01N 33/68, G01N 33/53

(54) **IMMUNOTHERAPEUTIC TREATMENT FOR INDUCING THE REGRESSION OF ATHEROSCLEROTIC PLAQUES**
IMMUNTHERAPEUTISCHE BEHANDLUNG ZUR INDUZIERUNG DER REGRESSION ATHEROSKLEROTISCHER PLAQUES
Therapie immunotherapeutique pour l'induction de la regression des plaques atherosclérotiques

(30) Priority: 02.09.2005 GB 0517878
(43) Date of publication of application: 11.06.2008
(60) Divisional application: 10075572.7
(73) Proprietor: BioInvent International AB, 223 70 Lund (SE)
(72) Inventor: CARLSSON, Roland c/o Anne-Christine Carlsson, SE-222 21 Lund (SE); NILSSON, Jan, S-240 13 Genarp (SE)
(74) Representative: Didmon, Mark
(86) International application number: PCT/EP2006/008594
(87) International publication number: WO 2007/025781

(56) References cited:
- WO-A-01/57226
- WO-A2-02/087611
- SCHIOPU A ET AL: "RECOMBINANT HUMAN ANTIBODIES AGAINST ALDEHYDE-MODIFIED APOLIPOPROTEIN B-100 PEPTIDE SEQUENCES INHIBIT ATHEROSCLEROSIS" CIRCULATION, AMERICAN HEART ASSOCIATION, DALLAS, TX, US, vol. 110, no. 14, 5 October 2004 (2004-10-05), pages 2047-2052, XP009048754 ISSN: 0009-7322 cited in the application
- NILSSON JAN ET AL: "Immunomodulation of atherosclerosis - Implications for vaccine development" ARTERIOSCLEROSIS THROMBOSIS AND VASCULAR BIOLOGY, vol. 25, no. 1, January 2005 (2005-01), pages 18-28, XP002420072 ISSN: 1079-5642 cited in the application
- FREDRIKSON GUNILLA NORDIN ET AL: "Inhibition of atherosclerosis in apoE-null mice by immunization with apoB-100 peptide sequences." ARTERIOSCLEROSIS, THROMBOSIS, AND VASCULAR BIOLOGY 1 MAY 2003, vol. 23, no. 5, 1 May 2003 (2003-05-01), pages 879-884, XP002420073 ISSN: 1524-4636 cited in the application
- BINDER C J ET AL: "Pneumococcal vaccination decreases atherosclerotic lesion formation: molecular mimicry between Streptococcus pneumoniae and oxidized LDL" NATURE MEDICINE, NATURE PUBLISHING GROUP, NEW YORK, NY, US, vol. 9, no. 6, June 2003 (2003-06), pages 736-743, XP002355525 ISSN: 1078-8956
- LIMA JOAO A C ET AL: "Statin-induced cholesterol lowering and plaque regression after 6 months of magnetic resonance imaging-monitored therapy" CIRCULATION, vol. 110, no. 16, 19 October 2004 (2004-10-19), pages 2336-2341, XP002420074 ISSN: 0009-7322
- STRÖM ASA ET AL: "Inhibition of injury-induced arterial remodelling and carotid atherosclerosis by recombinant human antibodies against aldehyde-modified apoB-100." ATHEROSCLEROSIS FEB 2007, vol. 190, no. 2, February 2007 (2007-02), pages 298-305, XP002420075 ISSN: 0021-9150

## Description

The present invention relates to immunotherapeutic methods for treatment of cardiovascular disease associated with atherosclerosis selected from myocardial infarction and stroke.

Atherosclerosis is a multifactorial disease developing preferentially in subjects presenting biochemical risk factors including smoking, hypertension, diabetes mellitus, hypercholesterolemia, elevated plasma low-density lipoprotein (LDL) and triglycerides, hyperfibrinogenemia and hyperglycemia, among others. Atherosclerosis is a chronic disease that causes a thickening of the innermost layer (the intima) of large and medium-sized arteries. It decreases blood flow and might cause ischemia and tissue destruction in organs supplied by the affected vessel. Atherosclerotic lesions develop over a number of decades in humans, leading to complications such as coronary and cerebral ischemic and thromboembolic diseases and myocardial and cerebral infarction.

Atherosclerosis is the major cause of cardiovascular disease including myocardial infarction, stroke and peripheral artery disease. Cardiovascular disease is the leading cause of morbidity and mortality in industrialised countries and progresses steadily in emerging countries, with coronary atherosclerosis being the main underlying pathology. Current therapy of atherosclerosis is not completely effective at preventing disease development and complication.

The disease is initiated by accumulation of lipoproteins, primarily LDL, in the extracellular matrix of the vessel. These LDL particles aggregate and undergo oxidative modification. Oxidised LDL is toxic and causes vascular injury. Atherosclerosis represents, in many respects, a response to this injury including inflammation and fibrosis.

High plasma levels of cholesterol, and in particular high levels of LDL are generally recognised as driving forces for development of atherosclerosis whereas high levels of high-density lipoprotein (HDL counteract development of atherosclerosis). HDL has consequently been called the good cholesterol while
LDL has been called the bad cholesterol. Simplified, LDL transports cholesterol to tissue while HDL absorbs cholesterol from tissue and transports it to the liver where it becomes degraded. Therapeutic strategies to reduce LDL and increase HDL are under development for treatment of atherosclerosis. One particularly interesting and promising strategy involves a mutated variant of HDL the so-called ApoA-1_{Milano}. This HDL variant is very efficient in transporting cholesterol from the tissue and animal experiments (Shah *et al,* 1998) and results from clinical trials (Nissen *et al,* 2003) have demonstrated that it can cause significant reduction of atherosclerotic plaque burden.

As summarised in the background of WO 02/080954, Palinski et at (1989) identified circulating autoantibodies against oxidised LDL in humans. This observation suggested that atherosclerosis might be an autoimmune disease caused by immune reactions against oxidised lipoproteins. At this time, several laboratories began searching for associations between antibody titers against oxidised LDL and cardiovascular disease.

Antibodies against oxidised LDL were found to be present in patients with cardiovascular disease as well as in healthy controls. This led researchers to investigate whether autoimmune reactions against oxidised LDL in the vascular wall played a role in the development of atherosclerosis by immunising animals against their own oxidised LDL. The idea behind this approach was that if autoimmune reactions against oxidised LDL are reinforced using classical immunisation techniques this would result in increased vascular inflammation and progression of atherosclerosis. To test this hypothesis rabbits were immunised with homologous oxidised LDL and then induced atherosclerosis by feeding the animals a high-cholesterol diet for 16 weeks (Ameli *et al,* 1996; Freigang *et al,* 1998). However, in contrast to the original hypothesis, immunisation with oxidised LDL had a protective effect reducing development of atherosclerosis with about 50%. Similar results were also obtained in a subsequent study in which the high-cholesterol diet was combined with vascular balloon-injury to produce a more aggressive plaque development (Nilsson *et al,* 1997). Taken together the available data suggested that there exist immune reactions that protect against the development of atherosclerosis and that these involve autoimmunity against oxidised LDL.

These observations suggested the possibility of developing an immune therapy or "vaccine" for treatment of atherosclerosis-based cardiovascular disease in man. One approach to do this would be to immunise an individual with his or her own LDL after it has been oxidised, for example by exposure to copper.

Palinski *et al,* (1995) and George *et al,* (1998) have shown that immunisation against oxidised LDL reduces the development of atherosclerosis. Similarly Zhou *et al,* (2001) demonstrated that antibodies reactive with epitopes found on oxidised forms of LDL protected against development of atherosclerosis in animal models.

WO 02/080954 reports that vaccination of animals prone to develop atherosclerosis with oxidised forms of certain peptides derived from apolipoprotein B-100 (ApoB-100) protects them from development of atherosclerosis. Previous experiments have demonstrated that antibodies that bind to oxidised epitopes present in LDL particles (Schipou *et al,* 2004) protect from development of atherosclerotic plaques in animal models. Moreover, radiolabelled forms of antibodies that bind to oxidised LDL can also be used for radioimmunodetection of atherosclerotic lesions in experimental animals (Tsimikas *et al,* 2000). An ¹²⁵Iodine labelled anti MDA lysine epitope antibody was used to detect plaque in mice and rabbits, and the injected antibody was found to localise to plaques in the aorta. This indicates that immune reactions against oxidised LDL may have a protecting effect.

Also, recombinant human antibodies raised against MDA-modified peptides derived from human ApoB-100 were shown to significantly inhibit plaque formation in appropriate animal models (Schiopu *et al,* (2004); WO 2004/030607). The underlying mechanisms for this effect on plaque build up is not known, but an effect on macrophage activation and inflammation was suggested (Schiopu *et al,* 2004). Oxidised LDL is known to activate macrophages leading to an accelerated inflammatory process that in turn drives atherosclerosis (Smith *et al*, 1995). However, there was no expectation, nor any experimental evidence, to suggest that an immune response against oxidised LDL, or administration of pre-made antibodies reactive against oxidised LDL, might result in the regression of pre-existing atherosclerotic lesions.

Previous work has also demonstrated that antibodies against oxidised peptides derived from ApoB-100 as well as antibodies against other oxidised LDL epitopes including phosphatidylcholine can prevent development of atherosclerotic plaques in experimental animals. (2004/030607; US6,716,410). Injection of human immunoglobulins containing natural anti-oxLDL antibodies reduced the atherosclerosis in ApoE deficient mice (Nicoletti *et al.,* 1998).

As far as we are aware, none of the authors of any of these publications appreciated that antibodies against oxidised LDL epitopes may cause regression of atherosclerotic plaques, nor did they suggest that such antibodies can be used in human patients for reduction of the atherosclerotic plaque burden.

Surprisingly and unexpectedly, we have now found that not only do antibodies against oxidised LDL epitopes interfere with plaque formation, they also induce regression of already-formed atherosclerotic plaques. Such antibodies have a potential to be used for therapy of advanced atherosclerosis to actively revert disease progression resulting in a reduced plaque burden.

Similarly, since specific antibody titers in mammals can be achieved either by passive or active immunisation, regression of pre-existing plaques can be obtained by either of the two approaches.

The present invention is described in the accompanying claims, and relates to the regression of pre-existing atherosclerotic plaques in a human individual who has a cardiovascular disease associated with atherosclerosis selected from myocardial infarction and stroke, by passive immunisation by administration of antibodies raised against oxidised epitopes of ApoB100 or an oxidised lipid epitope of LDL.

Also described herein but not claimed is immunotherapy by active immunisation by administration of oxidised epitopes of LDL.

By "regression of atherosclerotic plaques" we include the meaning of reducing the size and/or amount and/or extent of atherosclerotic plaques. Typically, regression of atherosclerotic plaques leads to a reduction in the area of the interior arterial surface covered by plaques. Thus by "regression of atherosclerotic plaques" we include reducing the overall plaque burden in the individual, as well as reducing the size of some, or all, of the individual atherosclerotic plaques. Regression of atherosclerotic plaques also leads to an increase in the vascular lumen contributing to increased blood flow.

Methods for measuring the size and/or amount and/or extent of atherosclerotic plaques in an individual are well known to the person of skill in the art and include angiography, vascular ultrasound, computer tomography and magnetic resonance imaging.

By "reducing the size and/or amount and/or extent" we include a reduction of about 1-25%, such as a reduction of about 1 or 2 or 3 or 4 or 5%, or a larger reduction of about 6 or 7 or 8 or 9 or 10%, or a reduction of 10-25%. More preferred is a larger reduction of 25-50%, or 50-75%, or more.

By reducing the area of the interior arterial surface covered by atherosclerotic plaques we include a reduction of about 1-25%, such as a reduction of about 1 or 2 or 3 or 4 or 5%, or a larger reduction of about 6 or 7 or 8 or 9 or 10%, or a reduction of 10-25%. More preferred is a larger reduction of 25-50%, or 50-75%, or more.

By an increase in the effective cross-section of the arterial vessel we include the meaning of an increase of 1-25%, such as an increase of about 1 or 2 or 3 or 4 or 5%, or a larger increase of about 6 or 7 or 8 or 9 or 10%, or an increase of 10-25%. More preferred is a larger increase of 25-50%, or 50-75%, or 75-100%. Most preferably, the effective cross-section of the arterial vessel is increased 2- or 3- or 4- or 5- or 10-fold, or more. Clearly, the extent of increase of cross-section of the arterial vessel is dependent upon the level of arterial blockage caused by atherosclerotic lesions prior to treatment.

The atherosclerotic plaques to be regressed are typically those in the aorta of the individual, but may also be found in other arterial sites in the patient like the femoral, carotid and coronary arteries.

The immunotherapy is directed against an oxidised epitope of ApoB100 or an oxidised lipid epitope of LDL but not on native LDL. Such epitopes can be determined using methods well known to a person of skill in the art and described in WO02/080954.

Oxidised LDL contains several different epitopes that can be recognised by antibodies. LDL may undergo oxidative and degrading changes through a wide variety of different chemical reactions. These include reactions caused by different types of modifications caused by the activity of oxygen, enzymes (e.g. myeloperoxidase), metal ions (e.g. Fe²⁺ and CU²⁺), free radicals and other types of chemical stress.

Some of the oxidised epitopes are found on the protein part of LDL (Yang *et al,* 2001) but others are modifications of lipids present in the LDL particle. Many oxidatively modified and biologically active phospholipids can be formed (Heery *at al*, 1995; Friedman *et al,* 2002; Watson *et al*, 1999). Polyunsaturated fatty acids are converted to fatty acid hydroperoxides, which rapidly form highly reactive products such as malondialdehyde and 4-hydroxynonenal (Smiley *et al*, 1991). These type of intermediate products can go on to form covalent Schiff base and Michael-type products with the lysine in ApoB-100 protein of LDL. Reactive aldehydes can also be found in fatty acids attached via the ester bonds in the phosphatidycholine moiety (Witztum & Berliner, 1998). Frequently found is phospholipid 1-palmitoyl-2-arachidoncylsn-glycero-3-phosphorylcholine (PAPC), a near terminal oxidation product that yields an aldehyde at a carbon of the sn-2 oxidized arachidonic acid, resulting in POVPC (1-palmitoyl-2-(5-oso)valeroyl-sn-glycero-3-phosphorylcholine). POVPC can react with lysine and also with amine-containing phospholipids such as phosphatidylethanolamine and phosphatidylserine. The end result is a variety of oxidised lipid-protein and oxidised lipid-lipid adducts. Some of these oxidations are driven by enzymes such as secretory phospholipase (Leitinger *et al,* 1999). Other enzyme-formed changes such as nitration and addition of HOCL are performed by myeloperoxidase (Carr *et al,* 2000). All neoepitopes are to be considered as immunogenic and biologically active (McIntyre *et al*, 1999; Esterbauer *et al*, 1991). Also cryptic epitopes that are a result of an oxidised modification of the LDL particle but are not oxidised themselves, like phosphorylcholine and fragments of proteins, are hallmarks of oxidised LDL particles and such epitopes are targetable by immunotherapy.

Recently the present inventors developed human antibodies from a recombinant antibody fragment library called n-CoDeR® that were directed against oxidised peptides derived from human ApoB-100 (WO02/080954). These peptide epitopes of ApoB-100, taken from Table 1 of WO02/080954, are listed in Table 1, below. The developed antibodies protected from development of atherosclerotic lesions in animal models (Schiopu et al, 2004; WO 2004/030607). The sequences of the anti-oxidised ApoB-100 antibodies disclosed in WO 2004/030607, especially IEI-A8, IEI-D8, IEI-E3, IEI-G8, KTT-B8 and KTT-D6. For the avoidance of doubt, each of the antibodies described in WO 2004/030607 and in Schiopu et al (2004) are examples of antibodies that may be used in the context of the present invention.

**Table 1**

| **PEPTIDE NAME** | **PEPTIDE SEQUENCE** | **SEQ ID NO:** |
|---|---|---|
| ***Category A. High IgG, MDA-difference*** | | |
| P.11. | FLDTVYGNCSTHFTVKTRKG | 1 |
| P.25. | PQCSTHILQSLKRVHANPLL | 2 |
| P.74 | VISIPRLQAEARSEILAHWS | 3 |

| ***Category B. High IgM, no MDA-difference*** | | |
|---|---|---|
| P 40. | KLVKEALKESQLPTVMDFRK | 4 |
| P 68. | LKFVTQAEGAKQTEATMTFK | 5 |
| P 94. | DGSLRHKFLDSNIKFSHVEK | 6 |
| P 99. | KGTYGLSCQRDPNTGRLNGE | 7 |
| P 100. | RLNGESNLRFNSSYLQGTNQ | 8 |
| P 102. | SLTSTSDLQSGIIKNTASLK | 9 |
| P 103. | TASLKYENYELTLKSDTNGK | 10 |
| P 105. | DMTFSKQNALLRSEYQADY'E | 11 |
| P 177. | MKVKIIRTIDQMQNSELQWP | 12 |

| ***Category C. High IgG, no MDA difference*** | | |
|---|---|---|
| P 143. | IALDDAKINFNEKLSQLQTY | 13 |
| P 210. | KTTKQSFDLSVKAQYKKNKH | 14 |

| ***Category D. NHS*/*AHP, IgG-ak > 2, MDA-difference*** | | |
|---|---|---|
| P1. | EEEMLENVSLVCPKDATRFK | 15 |
| P 129. | GSTSHHLVSRKSISAALEHK | 16 |
| P 148. | IENIDFNKSGSSTASWIQNV | 17 |
| P 162. | IREVTQRLNGEIQALELPQK | 18 |
| P 252. | EVDVLTKYSQPEDSLIPFFE | 19 |

| ***Category E. NHS*/*AHP, IgM-ak > 2, MDA-difference*** | | |
|---|---|---|
| P 301. | HTFLIYITELLKKLQSTTVM | 20 |
| P 30. | LLDIANYLMEQIQDDCTGDE | 21 |
| P 31. | CTGDEDYTYKIKRVIGNMGQ | 22 |
| P 32. | GNMGQTMEQLTPELKSSILK | 23 |
| P 33. | SSILKCVQSTKPSLMIQKAA | 24 |
| P 34. | IQKAAIQALRKMEPKDKDQE | 25 |
| P 100. | RLNGESNLRFNSSYLQGTNQ | 26 |
| P 107. | SLNSHGLELNADILGTDKIN | 27 |
| P 149. | WIQNVDTKYQIRIQIQEKLQ | 28 |
| P 169. | TYISDWWTLAAKNLTDFAEQ | 29 |
| P 236. | EATLQRIYSLWEHSTKNHLQ | 30 |

| ***Category F. NHS*/*AHP, IgG-ak < 0.5, no MDA-difference*** | | |
|---|---|---|
| P 10. | ALLVPPETEEAKQVLFLDTV | 31 |
| P 45. | IEIGLEGKGFEPTLEALFGK | 32 |
| P111. | SGASMKLTTNGRFREHNAKF | 33 |
| P 154. | NLIGDFEVAEKINAFRAKVH | 34 |
| P 199. | GHSVLTAKGMALFGEGKAEF | 35 |
| P 222. | FKSSVITLNTNAELFNQSDI | 36 |
| P 240. | FPDLGQEVALNANTKNQKIR | 37 |

| ***Category G. No level of IgG or IgM antibodies*** | | |
|---|---|---|
| P 2 | ATRFKHLRKYTNYQAQSSS | 38 |

As shown in Table 1 above, the peptides could be grouped into six categories with common characteristics:
Category A: Fragments that produce high levels of IgG antibodies to MDA-modified peptides (n=3).
Category B: Fragments that produce high levels of IgM antibodies, but no difference between native and MDA-modified peptides (n=9).
Category C: Fragments that produce high levels of IgG antibodies, but no difference between native and MDA-modified peptides (n=2).
Category D: Fragments that produce high levels of IgG antibodies to MDA-modified peptides and at least twice as much antibodies in the NHP-pool as compared to the AHP-pool (n=5).
Category E: Fragments that produce high levels of IgM antibodies to MDA-modified peptides and at least twice as much antibodies in the NHP-pool as compared to the AHP-pool (n=11).
Category F: Fragments that produce high levels of IgG antibodies, but no difference between intact and MDA-modified peptides but at least twice as much antibodies in the AHP-pool as compared to the NHP-pool (n=7).

It is appreciated that the peptide fragments of ApoB-100 may be made using protein chemistry techniques for example using partial proteolysis (either exolytically or endolytically), or by *de novo* synthesis. Alternatively the variants may be made by recombinant DNA technology. Suitable techniques for cloning, manipulation, modification and expression of nucleic acids, and purification of expressed proteins, are well known in the art and are described for example in Sambrook et al (2001), "Molecular Cloning, a Laboratory Manual", 3rd edition, Sambrook et al (eds), Cold Spring Harbor Laboratory Press, Cold Spring Harbor, NY, USA.

By "peptide" we include not only molecules in which amino acid residues are joined by peptide (-CO-NH-) linkages but also molecules in which the peptide bond is reversed. Such retro-inverso peptidomimetics may be made using methods known in the art, for example such as those described in Mezier *et al,* 1997. This approach involves making pseudopeptides containing changes involving the backbone, and not the orientation of side chains. At least for MHC class II and T helper cell responses, these pseudopeptides were shown to be useful. Retro-inverse peptides, which contain NH-CO bonds instead of CO-NH peptide bonds, are much more resistant to proteolysis. Similarly, the peptide bond may be dispensed with altogether provided that an appropriate linker moiety which retains the spacing between the Cα atoms of the amino acid residues is used; it is particularly preferred if the linker moiety has substantially the same charge distribution and substantially the same planarity of a peptide bond. It is also appreciated that the peptide may conveniently be blocked at its N- or C- terminus so as to help reduce susceptibility to exoproteolytic digestion.

The invention thus includes the use of the oxidised peptide epitopes of ApoB-100 listed in Table 1, or an active fragment of one or more of these peptides, for immunotherapy against oxidised LDL to induce regression of pre-existing atherosclerotic plaques, by passive immunisation i.e. administration of antibodies raised against these oxidised epitopes.

Also described herein but not claimed is active immunisation in which, for vaccination purposes, ApoB-100 peptides can be administered in either oxidised or unoxidised form (because they seem to become oxidised when administered *in vivo*).

For the avoidance of doubt, in the context of this invention, the antibodies for passive immunisation all bind to oxidised LDL.

By a "fragment" of a peptide epitope of ApoB-100 listed in Table 1 we mean at least six consecutive amino acids of the given sequence. Thus a fragment may include 6 or 7 or 8 or 9 or 10 or 11 or 12 or 13 or 14 or 15 or 16 or 17 or 18 or 19 consecutive amino acids of the given sequence. An "active" fragment is one which, when oxidised, can be used to raise antibodies that induce regression of atherosclerotic plaques in an individual by either active or passive immunotherapy. Methods for determining whether any particular fragment of the peptide epitopes listed in Table 1 are active fragments as defined are provided in the Examples.

In the present invention, the at least one antibody selectively binds to an oxidised epitope of ApoB-100 or an oxidised lipid epitope of LDL.

Also described herein but not claimed is a method of inducing regression of atherosclerotic plaques in an individual in need thereof, the method comprising administering to the individual at least one antibody that selectively binds to an oxidised epitope of LDL.

The invention relates to the use of at least one antibody that selectively binds to an oxidised epitope ApoB-100 or an oxidised lipid epitope of LDL in the preparation of a medicament that induces regression of atherosclerotic plaques in a human individual who has a cardiovascular disease associated with atherosclerosis selected from myocardial infarction and stroke.

Also described herein but not claimed is administration of an antibody that selectively binds to an oxidised epitope of LDL which comprises administering to the individual a polynucleotide encoding said antibody molecule.

Methods of delivering polynucleotides to a patient are well known to a person of skill in the art and include the use of immunoliposomes, viral vectors (including vaccinia, modified vaccinia, and adenovirus), and by direct delivery of DNA, e.g. using a gene-gun and electroporation. For example, Svensson *et al*, 1999, describes the delivery of recombinant genes to cardiomyocytes by intramyocardial injection or intracoronary infusion of cardiotropic vectors, such as recombinant adeno-associated virus vectors, resulting in transgene expression in murine cardiomyocytes *in vivo.* Melo *et al* (2004) review gene and cell-based therapies for heart disease. An alternative preferred route of administration is via a catheter or stent. Stents represent an attractive alternative for localized gene delivery, as they provide a platform for prolonged gene elution and efficient transduction of opposed arterial walls. This gene delivery strategy has the potential to decrease the systemic spread of the viral vectors and hence a reduced host immune response. Both synthetic and naturally occurring stent coatings have shown potential to allow prolonged gene elution with no significant adverse reaction (Sharif *et al,* 2004).

A polynucleotide encoding an antibody molecule can be operatively linked to targeting and/or regulatory sequences that direct expression of the antibody to the arteries, and preferably the arterial walls. Thus the polynucleotide allows generation of specific antibodies within an affected individual. Suitable targeting and regulatory sequences are known to the skilled person.

In an embodiment that is described herein but not claimed, it may be desirable that expression of the polynucleotide is directly or indirectly (see below) under the control of a promoter that may be regulated, for example by the concentration of a small molecule that may be administered to the patient when it is desired to activate or, more likely, repress (depending upon whether the small molecule effects activation or repression of the said promoter) expression of the antibody from the polynucleotide. This may be of particular benefit if the expression construct is stable, i.e. capable of expressing the antibody (in the presence of any necessary regulatory molecules), in the cell for a period of at least one week, one, two, three, four, five, six, eight months or one or more years. Thus the polynucleotide may be operatively linked to a regulatable promoter. Examples of regulatable promoters include those referred to in the following papers: Rivera et al (1999) Proc Natl Acad Sci USA 96(15), 8657-62 (control by rapamycin, an orally bioavailable drug, using two separate adenovirus or adeno-associated virus AAV) vectors, one encoding an inducible human growth hormone (hGH) target gene, and the other a bipartite rapamycin-regulated transcription factor); Magari et al (1997) J Clin Invest 100(11), 2865-72 (control by rapamycin); Bueler (1999) Biol Chem 380(6), 613-22 (review of adeno-associated viral vectors); Bohl et al (1998) Blood 92(5), 1512-7 (control by doxycycline in adeno-associated vector); Abruzzese et al (1996) J Mol Med 74(7), 379-92 (review of induction factors, e.g. hormones, growth factors, cytokines, cytostatics, irradiation, heat shock and associated response elements).

In a preferred embodiment, the antibody molecule is an antibody raised against an oxidised epitope ApoB-100 or an oxidised lipid epitope of LDL, such as those listed in Table 1 and described in WO 02/080954. As described in WO 02/080954, peptides may be oxidised by exposure to a variety of agents such as iron, oxygen, copper, myeloperoxidase, phospholipase, hypochlorous acid, or by malone dealdehyde (MDA) modification, to mimic the different modifications of the amino acids that may occur during oxidation of LDL. Alternatively, other methods known in the art may be employed to oxidise the epitopes of LDL.

Generation of human antibodies reactive with MDA-modified ApoB-100 derived peptides has been described in WO 02/090854 and in Schiopu et al (2004). As discussed in detail below, human, or human-like antibodies can also be generated using other technologies well known in the art, including immunisation of mice transgenic in the human immunoglobulin locus, or by humanisation of e.g. murine antibodies with the desired specificity.

By an antibody that "selectively binds to an oxidised epitope of ApoB-100 or an oxidised lipid epitope of LDL" we mean that the antibody molecule binds the oxidised epitope of ApoB-100 or an oxidised lipid epitope of LDL with a greater affinity than for unoxidised LDL. Preferably, the antibody binds the oxidised epitope of ApoB-100 or an oxidised lipid epitope of LDL with at least 1.5, or at least 2, or at least 5, or at least 10 or at least 50 times greater affinity than for unoxidised LDL. More preferably, the antibody molecule binds the oxidised epitope of ApoB-100 or an oxidised lipid epitope of LDL with at least 100, or at least 1,000, or at least 10,000 times greater affinity than for unoxidised LDL. Such binding may be determined by methods well known in the art, such as one of the Biocore® systems. Preferably, the antibody molecule selectively binds an oxidised epitope of ApoB-100 or an oxidised lipid epitope of LDL and does not bind unoxidised LDL.

It is preferred if the antibodies have an affinity for their target epitope of at least 10⁻⁸M, although antibodies with higher affinities may be even more preferred.

The protective effects of humoral immunity are known to be mediated by a family of structurally related molecules called antibodies. Antibodies initiate their biological activity by binding to antigens. Antibody binding to antigens is generally specific for one antigen and the binding is usually of high affinity. Antibodies are produced by B-lymphocytes. Blood contains many different antibodies, each derived from a clone of B-cells and each having a distinct structure and specificity for antigen. Antibodies are present on the surface of B-lymphocytes, in the plasma, in interstitial fluid of the tissues and insecretory fluids such as saliva and mucous on mucosal surfaces. All naturally-occurring antibodies are similar in their overall structure, accounting for certain similarities in physico-chemical features such as charge and solubility. All antibodies have a common core structure of two identical light chains, each about 24 kilo Daltons, and two identical heavy chains of about 55-70 kilo Daltons each. One light chain is attached to each heavy chain, and the two heavy chains are attached to each other. Both the light and heavy chains contain a series of repeating homologous units, each of about 110 amino acid residues in length which fold independently in a common globular motif, called an immunoglobulin (Ig) domain. The region of an antibody formed by the association of the two heavy chains is hydrophobic. Antibodies, and especially monoclonal antibodies, are known to cleave at the site where the light chain attaches to the heavy chain when they are subjected to adverse physical or chemical conditions. Because antibodies contain numerous cysteine residues, they have many cysteine-cysteine disulfide bonds. All Ig domains contain two layers of beta-pleated sheets with three or four strands of anti-parallel polypeptide chains.

Despite their overall similarity, antibody molecules can be divided into a small number of distinct classes and subclasses based on physicochemical characteristics such as size, charge and solubility, and on their behaviour in binding to antigens. In humans, the classes of antibody molecules are: IgA, IgD, IgE, IgG and IgM, and members of each class are said to be of the same isotype. IgA and IgG isotypes are further subdivided into subtypes called IgAl, IgA2 and IgG1, IgG2, IgG3 and IgG4. The heavy chains of all antibodies in an isotype share extensive regions of amino acid sequence identity, but differ from antibodies belonging to other isotypes or subtypes. IgG, IgE and IgD circulate as monomers, whereas secreted forms of IgA and IgM are dimers or pentamers, respectively, stabilised by the J chain. Some IgA molecules exist as monomers or trimers.

By "antibody" or "antibody molecule" we include not only whole immunoglobulin molecules as described above but also fragments thereof such as Fab, F(ab')₂, Fv and other fragments thereof that retain the antigen-binding site. Similarly the term antibody includes genetically engineered derivatives of antibodies such as single chain Fv molecules (scFv) and domain antibodies (dAbs). The term also includes antibody-like molecules which may be produced using phage-display techniques or other random selection techniques for molecules which bind to oxidised LDL or to specified regions of it. Thus, the term antibody includes all molecules which contain a structure, preferably a peptide structure, which is part of the recognition site (ie the part of the antibody that binds or combines with the epitope or antigen) of a natural antibody.

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci USA 81, 6851-6855). That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter δ Milstein (1991) Nature 349, 293-299.

By "ScFv molecules" we mean molecules wherein the V_{H} and V_{L} partner domains are linked via a flexible oligopeptide. Engineered antibodies, such as ScFv antibodies, can be made using the techniques and approaches described in J. Huston et al, (1988) "Protein engineering of antibody binding sites: recovery of specific activity in an anti-digoxin single chain Fv analogue produced in E. coli", Proc. Natl. Acad. Sci, USA, 85, pp.5879-5883, and in A. Pluckthun, (1991) "Antibody engineering; Advances from use of E. coli expression systems", Bio/technology 9(6): 545-51.

The advantages of using antibody fragments, rather than whole antibodies, are several-fold. The smaller size of the fragments may lead to improved pharmacological properties, such as better penetration to the target site. Effector functions of whole antibodies, such as complement binding, are removed. Fab, Fv, ScFv and dAb antibody fragments can all be expressed in and secreted from *E. coli,* thus allowing the facile production of large amounts of the fragments.

Whole antibodies, and F(ab')₂ fragments are "bivalent". By "bivalent" we mean that the antibodies and F(ab')₂ fragments have two antigen combining sites. In contrast, Fab, Fv, ScFv and dAb fragments are monovalent, having only one antigen combining site.

It is preferred if the antibody is a monoclonal antibody. In some circumstance, particularly if the antibody is going to be administered repeatedly to a human patient, it is preferred if the monoclonal antibody is a human monoclonal antibody or a humanised monoclonal antibody.

Suitable monoclonal antibodies which are reactive as described herein may be prepared by known techniques, for example those disclosed in "Monoclonal Antibodies; A manual of techniques ", H Zola (CRC Press, 1988) and in *"*Monoclonal Hybridoma Antibodies: Techniques and Application ", SGR Hurrell (CRC Press, 1982).

The antibodies may be given desired properties concerning e.g. specificity and cross-reactivity, isotype, affinity and plasma half-life. The possibility to develop antibodies with predetermined properties became apparent already with the advent of the monoclonal antibody technology (Milstein and Köhler, 1975 Nature, 256: 495-7). This technology used murine hybridoma cells producing large amounts of identical, but murine, antibodies. In fact, a large number of preclinical, and also clinical trials were started using murine monoclonal antibodies for treatment of e.g. cancers. However, due to the fact that the antibodies were of non-human origin the immune system of the patients recognised them as foreign and developed antibodies to them. As a consequence the efficacy and plasma half-lives of the murine antibodies were decreased, and often side effects from allergic reactions, caused by the foreign antibody, prevented successful treatment.

To solve these problems several approaches to reduce the murine component of the specific and potentially therapeutic antibody were taken. The first approach comprised technology to make so called chimeric antibodies where the murine variable domains of the antibody were transferred to human constant regions resulting in an antibody that was mainly human *(*Neuberger et al, 1985, Nature 314: 268-70; Neuberger et al, 1998, 8th International Biotechnology Symposium Part 2, 792-799).

A further refinement of this approach was to develop humanised antibodies where the regions of the murine antibody that contacted the antigen, the Complementarity Determining Regions (CDRs) were transferred to a human antibody framework. Such antibodies are almost completely human and seldom cause any harmful antibody responses when administered to patients. Several chimeric or humanised antibodies have been registered as therapeutic drugs and are now widely used within various indications (Borrebaeck & Carlsson, 2001, Curr. Opin. Pharmacol. 1: 404-408).

It is preferred if the antibody is a humanised antibody. Suitably prepared non-human antibodies can be "humanised" in known ways, for example by inserting the CDR regions of mouse antibodies into the framework of human antibodies. Humanised antibodies can be made using the techniques and approaches described in Verhoeyen et al (1988) Science, 239, 1534-1536, and in Kettleborough et al, (1991) Protein Engineering, 14(7), 773-783.

Completely human antibodies may be produced using recombinant technologies. Typically large libraries comprising billions of different antibodies are used. In contrast to the previous technologies employing chimerisation or humanisation of e.g. murine antibodies this technology does not rely on immunisation of animals to generate the specific antibody. Instead the recombinant libraries comprise a huge number of pre-made antibody variants wherein it is likely that the library will have at least one antibody specific for any antigen. Thus, using such libraries, an existing antibody having the desired binding characteristics can be identified. In order to find the good binder in a library in an efficient manner, various systems where phenotype i.e. the antibody or antibody fragment is linked to its genotype i.e. the encoding gene have been devised. The most commonly used such system is the so called phage display system where antibody fragments are expressed, displayed, as fusions with phage coat proteins on the surface of filamentous phage particles, while simultaneously carrying the genetic information encoding the displayed molecule (McCafferty et al, 1990, Nature 348: 552-554). Phage displaying antibody fragments specific for a particular antigen may be selected through binding to the antigen in question. Isolated phage may then be amplified and the gene encoding the selected antibody variable domains may optionally be transferred to other antibody formats, such as e.g. full length immunoglobulin, and expressed in high amounts using appropriate vectors and host cells well known in the art.

The format of displayed antibody specificities on phage particles may differ. The most commonly used formats are Fab (Griffiths et al, 1994. EMBO J. 13: 3245-3260) and single chain (scFv) (Hoogenboom et al, 1992, J Mol Biol. 227: 381-388) both comprising the variable antigen binding domains of antibodies. The single chain format is composed of a variable heavy domain (V_{H}) linked to a variable light domain (V_{L}) via a flexible linker (US 4,946,778). Before use as a therapeutic agent, the antibody may be transferred to a soluble format e.g. Fab or scFv and analysed as such. In later steps the antibody fragment identified to have desirable characteristics may be transferred into yet other formats such as full length antibodies.

Recently a novel technology for generation of variability in antibody libraries was presented (WO 98/32845; Soderlind et al (2000) Nature BioTechnol. 18:852-856). Antibody fragments derived from this library all have the same framework regions and only differ in their CDRs. Since the framework regions are of germline sequence the immunogenicity of antibodies derived from the library, or similar libraries produced using the same technology, are expected to be particularly low (Soderlind *et al,* 2000). This property is of great value for therapeutic antibodies, reducing the risk that the patient forms antibodies to the administered antibody, thereby reducing risks for allergic reactions, the occurrence of blocking antibodies, and allowing a long plasma half-life of the antibody.

Thus, when developing therapeutic antibodies to be used in humans, modern recombinant library technology (Soderlind et al, 2001, Comb. Chem. & High Throughput Screen. 4: 409-416) is now used in preference to the earlier hybridoma technology.

It is appreciated that the peptides identified in WO 02/080954 and listed in Table 1 may be used as antigens for generation of fully human antibodies with predetermined properties, which may be particularly relevant as therapeutic antibodies for inducing regression of atherosclerotic plaques. The resulting fully human antibodies are not expected to generate any undesired immunological reaction when administered into patients.

Certain preferred antibodies include those that bind to the oxidised ApoB-100 epitopes or oxidised lipid epitopes of LDL of P45 (amino acid residues 661-680) and/or P143 (amino acid residues 2131-2150). Antibodies having this binding specificity include IEI-I3 (Schiopu *et al* 2004) and 2D03 (described in Example 2).

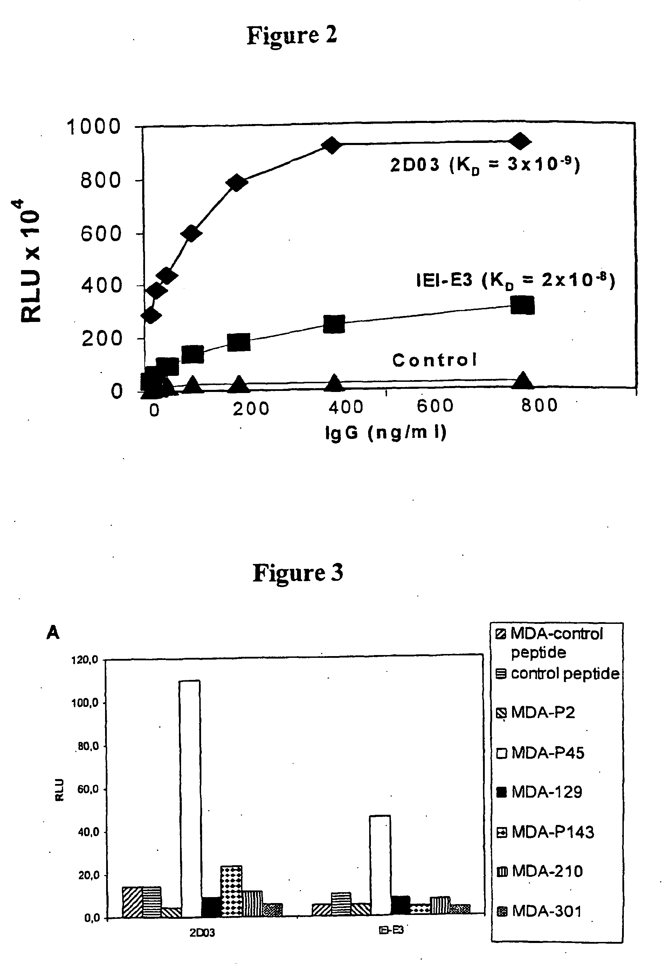

Antibody IEI-E3 has previously been shown to significantly inhibit development of plaque in an animal model (Schiopu *et al,* 2004). We have now shown (see Examples) that it can induce regression of atherosclerotic plaques. We have also demonstrated that other anti-oxidised LDL antibodies with a different specificity i.e. 2D03, LDO-D4 and KTT-B8, but not the control antibody FITC-8, could all significantly reduce the level of plaque in aorta compared to the plaque area seen in the animals before the start of treatment. The CDR sequences of the antibodies are listed in Table 2. The V_{H} and V_{L} sequences of antibodies IEI-E3 and KTT-B8 are given in Figure 3 of WO 2004/030607.

**Table 2. Amino acid sequences of CDRs found in anti-oxLDL antibodies**

| | **2D03** | **LDO-D4** | **IEI-E3** | **KTT-B8** |
|---|---|---|---|---|
| **H1** | FSNAWMSWVRQAPG (SEQ ID NO:39) | FSNAWMSVWRQAPG (SEQ ID NO:45) | FSDYYMSWVRQAPG (SEQ ID NO:51) | FSSYAMSWVRQAPG (SEQ ID NO:57) |
| **H2** | SSISVGGHRTYYADSVKGR (SEQ ID NO:40) | SSISTSSNYIYYADSVKGR (SEQ ID NO:46) | SGVSWNGSRTHYADSVKGR (SEQ ID NO:52) | SSISSSGRFIYYADSMICGR (SEQ ID NO:58) |
| **H3** | ARIRVGPSGGAFDY (SEQ ID NO:41) | ARVKKYSSGWYSNYAFDI (SEQ ID NO:47) | ARAARYSYYYYGMDV (SEQ ID NO:53) | TRLRRGSYFWAFDI (SEQ ID NO:59) |
| **L1** | CSGSNTNIGKNYVS (SEQ ID NO:42) | CSGSSSSIGNNFVS (SEQ ID NO:48) | CSGSSSNIGNNAVN (SEQ ID NO:54) | CSGSSSNIGGESVS (SEQ ID NO:60) |
| **L2** | ANSNRPS (SEQ ID NO:43) | DNNKRPS (SEQ ID NO:49) | GNDRRPS (SEQ ID NO:55) | SNNQRPS (SEQ ID NO:61) |
| **L3** | CASWDASLNGWV (SEQ ID NO:44) | CAAWDDSLNGWV (SEQ ID NO:50) | CQTWGTGRGV (SEQ ID NO:56) | CAAWDDSLNGWV (SEQ ID NO:62) |

Also described herein but not claimed is immunotherapy comprising administering to the individual at least one oxidised epitope of LDL. The at least one oxidised epitope of LDL acts to induce an immune response against the oxidised epitope so as to induce regression of atherosclerotic plaques in an individual. In other words, the at least one oxidised epitope of LDL acts as a vaccine to raise an immune response that induces regression of atherosclerotic plaques in the vaccinated individual.

Thus, also described herein but not claimed is a method of inducing regression of atherosclerotic plaques in an individual in need thereof, the method comprising administering to the individual at least one oxidised epitope of LDL.

Also described herein but not claimed is the use of at least one oxidised epitope of LDL in the preparation of a medicament for inducing regression of atherosclerotic plaques in an individual.

As defined in the accompanying claims, the invention relates to a human individual, who has a cardiovascular disease associated with atherosclerosis selected from myocardial infarction and stroke. Also described herein but not claimed is an individual which is a mammal such as a horse, cow, sheep, pig, camel, dog or cat.

The human individual is a patient who has a cardiovascular disease associated with atherosclerosis selected from myocardial infarction and stroke. Those diseases are medically linked to atherosclerosis in that they are a consequence of atherosclerotic lesions. Another cardiovascular disease associated with atherosclerosis but not claimed herein is coronary artery disease.

Whether or not a particular patient is one who is expected to benefit from treatment may be determined by the physician.

It is appreciated that since the methods and uses of the invention lead to regression of pre-existing atherosclerotic plaques, the invention includes reducing the risk of a cardiovascular disease associated with atherosclerosis selected from myocardial infarction and stroke in a patient who is at risk of developing said cardiovascular diseases due to the presence of the atherosclerotic plaques.

The patient who is at risk of said cardiovascular diseases associated with atherosclerosis may be one who has blood cholesterol levels that are likely to cause or exacerbate said cardiovascular diseases or dysfunction.

A patient may be at risk of developing coronary heart disease because of multiple risk factors (including obesity, smoking, hypertension, diabetes mellitus and a family history of premature coronary heart disease); one with a familial condition characterised by very high plasma concentrations of cholesterol and/or triglycerides; one with hyperlipidemia not secondary to underlying diseases (such as hypothyroidism, nephritic syndrome, hepatic disease or alcoholism); one with elevated LDL-cholesterol; or one under dietary hypolipidemic intervention (complementary treatment).

It is further appreciated that since the uses of the invention lead to reduction in the size of pre-existing atherosclerotic plaques, the invention is particularly useful for treading patients with advanced or severe atherosclerosis, and advanced or severe forms of the cardiovascular disease associated with atherosclerosis selected from myocardial infarction and stroke.

Also described herein but not claimed is the prior step of determining the size and/or amount and/or extent of atherosclerotic plaques in the individual. This may be done to assess whether the individual is in need of treatment to reduce his atherosclerotic plaque burden, or to provide a baseline measurement to assess the efficacy of such treatment, or for both purposes.

Thus described herein but not claimed is a method of identifying a patient with an atherosclerotic plaque burden in need of reduction, and subsequently administering to the individual at least one antibody that selectively binds to an oxidised epitope of LDL, or at least one oxidised epitope of LDL.

It is appreciated that an atherosclerotic plaque burden in need of reduction may be due to the size and/or extent of the overall plaque burden. Additionally or alternatively, this could be due to the nature of the plaques, for example, how unstable they are.

The invention may also be considered to include the use of at least one antibody that selectively binds to an oxidised epitope of ApoB-100 or an oxidised lipid epitope of LDL, in the preparation of a medicament for inducing regression of atherosclerotic plaques in an individual who has been assessed as being in need of such treatment by measuring the size and/or amount and/or extent of atherosclerotic plaques in the individual.

Also described herein but not claimed is the subsequent step of determining the size and/or amount and/or extent of atherosclerotic plaques in the patient after the administration of the at least one antibody that selectively binds to an oxidised epitope of ApoB-100 or an oxidised lipid epitope of LDL, so as to assess the efficacy of the treatment in comparison with a baseline measurement taken prior to treatment.

The dose of the at least one antibody that selectively binds to an oxidised epitope of ApoB-100 or an oxidised lipid epitope of LDL administered to the patient will typically be determined by the physician based on considerations such the atherosclerotic condition of the patient to be treated, other treatment agents to be administered, the age, sex and size of the patient, and so on.

Statins (inhibitors of 3-hydroxy-3-methylglutaryl-coenzyme A (HMGCoA) reductase) have proved to be effective in preventing acute cardiovascular events by reducing plasma cholesterol content (and by additional mechanisms yet to be clarified). Uses as defined in the claims involving administration of a statin in conjunction with at least one antibody that selectively binds to an oxidised epitope of ApoB-100 or an oxidised lipid epitope of LDL may be a useful means of treatment to supplement the regression of atherosclerotic plaques. Preferably the two dosage regimes are chosen to have a synergistic effect.

Described herein but not claimed is a method of combating a cardiovascular disease associated with atherosclerosis in an individual in need thereof, the method comprising:
inducing the regression of atherosclerotic plaques in an individual by administering to the individual: (a) at least one antibody molecule that selectively binds to an oxidised epitope of LDL, or (b) at least one oxidised epitope of LDL; and
administering a statin to the individual.

Also described herein but not claimed is the use of at least one antibody molecule as defined herein and a statin in the preparation of a medicament for combating a cardiovascular disease associated with atherosclerosis by inducing the regression of atherosclerotic plaques in an individual who has a cardiovascular disease associated with atherosclerosis selected from myocardial infarction and stroke, comprising the step of administering the at least one antibody molecule to the individual after the statin has been administered.

Also described herein but not claimed is the use of at least one antibody molecule as defined herein and a statin in the preparation of a medicament for combating a cardiovascular disease associated with atherosclerosis in an individual who has a cardiovascular disease associated with atherosclerosis selected from myocardial infarction and stroke, comprising the step of administering the statin to the individual after the at least one antibody molecule has been administered.

A further aspect of the invention provides the use of at least one antibody molecule as defined herein and a statin, in the preparation of a medicament for combating a cardiovascular disease associated with atherosclerosis in a human individual who has a cardiovascular disease associated with atherosclerosis selected from myocardial infarction and stroke by inducing the regression of atherosclerotic plaques.

Also described herein but not claimed is a pharmaceutical formulation comprising: at least one antibody molecule that selectively binds to an oxidised epitope of ApoB-100 or an oxidised lipid epitope of LDL and a statin, in admixture with a pharmaceutically-acceptable adjuvant, diluent or carrier.

Also described herein but not claimed is a kit of parts comprising components:
at least one antibody molecule that selectively binds to an oxidised epitope of ApoB-100 or an oxidised lipid epitope of LDL, and
a statin,
wherein the components are each provided in a form that is suitable for administration in conjunction with the other.

By "in conjunction" we include the meaning that the components may be suitable for simultaneous or combined administration to the patient. However, since the components may need be administered by different routes or at different rates, by "in conjunction" we also include the meaning of consecutive administration or separate administration within the same treatment regime.

Suitable statins include atorvastatin, cerivastatin, fluvastatin, lovastatin, mevastatin, pravastatin, rosuvastatin and simvastatin.

Also described herein but not claimed is an antibody coupled to an anti-inflammatory agent. Suitable anti-inflammatory agents include steroidal compounds such as dexamethasone, betamethasone, prednisone, prednisolone, triamcinolone, hydrocortisone, alclometasone, amcinonide, diflorasone, etc. as well as non-steroidal anti-inflammatories. Methods for coupling compounds, such as the anti-inflammatory agents listed above, to antibodies are well known in the art.

Also described herein but not claimed is an *ex vivo* method of identifying an antibody that induces regression of atherosclerotic plaques in an individual, the method comprising:
providing an antibody that selectively binds to an oxidised epitope of ApoB-100 or an oxidised lipid epitope of LDL and
testing it in an assay for atherosclerotic plaque regression, wherein regression of atherosclerotic plaques in the assay indicates that the antibody is one that induces regression of atherosclerotic plaques.

Also described herein but not claimed is an atherosclerotic plaque regression assay which is an *in vivo* assay such as the animal models described in Example 2 or 4.

Preferably, the tested antibody has been isolated from a human antibody fragment library as described above.

Identified antibody fragments with desired characteristics of atherosclerotic plaque regression may then be rebuilt into other antibody configurations, e.g. full length human immunoglobulin, to be used for therapeutic purposes.

Also described herein but not claimed is an *in vitro* method of identifying an agent that induces regression of atherosclerotic plaques in an individual, the method comprising:
providing an antibody that selectively binds to an oxidised epitope of ApoB-100 or an oxidised lipid epitope of LDL,
administering the antibody to an atherosclerotic plaque, and
determining whether said antibody induces regression of the atherosclerotic plaques,
wherein regression of the atherosclerotic plaques indicates that the antibody is one that induces regression of atherosclerotic plaques.

Described herein but not claimed is an *in vivo* method, comprising an animal model of atherosclerosis, such as described in Example 2 or 4. Alternatively, the method may be employed in the context of a clinical trial of the agent, in which case the individual may be a human individual who has atherosclerotic plaques.

Described herein but not claimed is an antibody comprising:
At least one complementarity determining region (CDR) that has the amino acid sequence of the corresponding CDR of antibody 2D03 as shown in Table 2, or
at least one CDR that has the sequence of the corresponding CDR of antibody LDO D4 as shown in Table 2.

The said antibody may have two or three or four or five CDRs that have the sequence of the corresponding CDRs of antibody 2D03 or of LDO D4.

The said antibody may have three or four CDRs that have the sequence of the corresponding CDRs of antibody 2D03 or of LDO D4, it is preferred if the antibody has all three heavy chain or all three light chain CDRs that have the sequence of the corresponding CDRs of antibody 2D03 or LDO D4.

Also described herein but not claimed is an antibody comprising:
three light chain CDRs that have the sequence of the corresponding three light chain CDRs of antibody 2D03, or
three heavy chain CDRs that have the sequence of the corresponding three heavy chain CDRs of antibody 2D03, or
three light chain CDRs that have the sequence of the corresponding three light chain CDRs of antibody LDO D4, or
three heavy chain CDRs that have the sequence of the corresponding three heavy chain CDRs of antibody LDO D4.

Also described herein but not claimed is an antibody comprising three light chain CDRs and three heavy chain CDRs that have the sequence of the corresponding CDRs of antibody 2D03, or three light chain CDRs and three heavy chain CDRs that have the sequence of the corresponding CDRs of antibody LDO D4.

If the antibody does not comprise all six CDRs that have the sequence of the corresponding CDRs of antibody 2D03 or LDO D4, it is preferred if some or all of the 1, 2, 3, 4 or 5 "non-identical" CDRs comprise a variant of the sequence of the corresponding CDRs of antibody 2D03 or LDO D4. By "a variant" we include the meaning that the variant has at least 50% sequence identity with the sequence of the corresponding CDR, more preferably at least 70%, yet more preferably at least 80% or at least 90% or at least 95%. Most preferably, the variant has 96% or 97% or 98% or 99% sequence identity with the sequence of the corresponding

CDR of antibody 2D03 or LDO D4. Typically the "variant" CDR sequence has 5 or 4 or 3 or 2 or only 1 amino acid residue difference from the sequence of the corresponding CDR of antibody 2D03 or LDO D4.

Also described herein but not claimed is antibody 2D03, and antibody LDO D4.

Also described herein but not claimed is an antibody that selectively binds to the oxidised-LDL epitope that is selectively bound by antibody 2D03 or by antibody LDO D4. Methods for determining whether any given antibody selectively binds to the oxidised-LDL epitope that is selectively bound by antibody 2D03 or by antibody LDO D4 are well known to the person of skill in the art.

The invention also includes the use of an antibody in the preparation of a medicament for inducing regression of atherosclerotic plaques as defined in the claims.

The listing or discussion of a prior-published document in this specification should not necessarily be taken as an acknowledgement that the document is part of the state of the art or is common general knowledge.

The invention will now be described in more detail by reference to the following Examples and Figures.

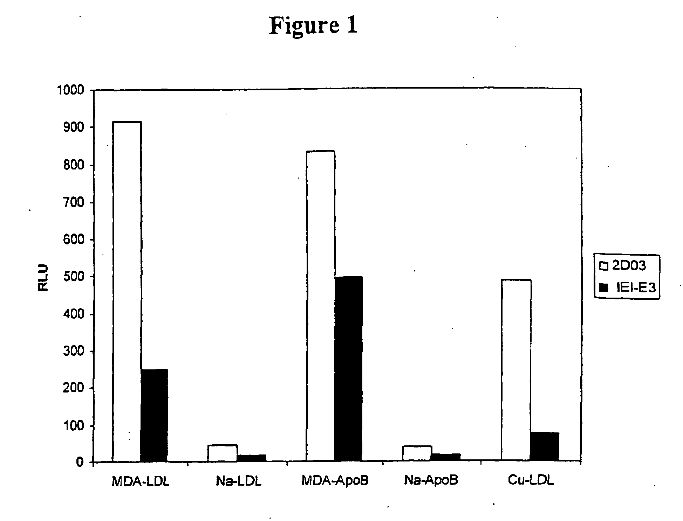

**Figure 1****.** Luminescence ELISA demonstrating binding of 2D03 and IEI-E3 antibodies to oxidised and native forms of LDL and ApoB-100. The prefix MDA represents MDA-modified forms of LDL and ApoB-100 whereas Na represents the native forms of these entities. Cu-LDL represents copper-oxidised LDL. Binding was detected with anti human IgG conjugated to peroxidise (RLU = relative luminescence units).
**Figure 2****.** ELISA based analysis of binding of 2D03 and IEI-E3 antibodies to MDA-modified human ApoB-100. Control corresponds to binding of FITC-8 antibody to the same antigen. Affinities were determined by Biacore analysis.
**Figure 3****.** Binding of single chain fragment (scFv) of IEI-E3 and 2D03 to a number of different MDA modified antigens. P2 (amino acids [aa] 16-31); P45 (aa 661-680); P129 (aa 1921-1940); P143 (aa 2131-2150); P210 (aa 3136-3155); and P301 (aa 4502-4521) are peptides corresponding to the specified regions of human Apo B-100 sequence. The control peptide was a non-relevant lysine-containing peptide (MDA-modified, black). Data are plotted as signal/10⁵. Abbreviations are as defined in text.

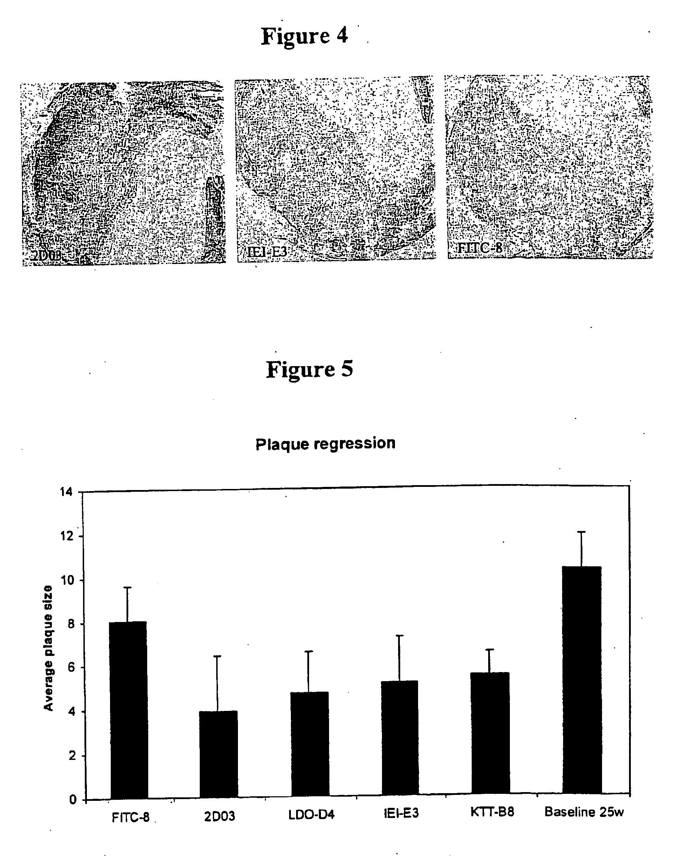

**Figure 4****.** Immunohistochemical staining of human plaques with 2D03, IEI-E3 and a control FITC-8 antibody. Bound antibody was detected with a horseradish peroxidase conjugated anti antibody and diamino benzidine staining.
**Figure 5****.** Plaque area in the descending aorta of atherosclerotic ApoBec mice treated with 2D03 and IEI-E3, LDO-D4 and KTT B8 antibodies which are specific for oxidised LDL. The isotype matched FITC-8 antibody was used as a control. Plaque area was assessed by Oil Red O staining. The values are expressed as percentage of total plaque area per total area of the descending aorta.

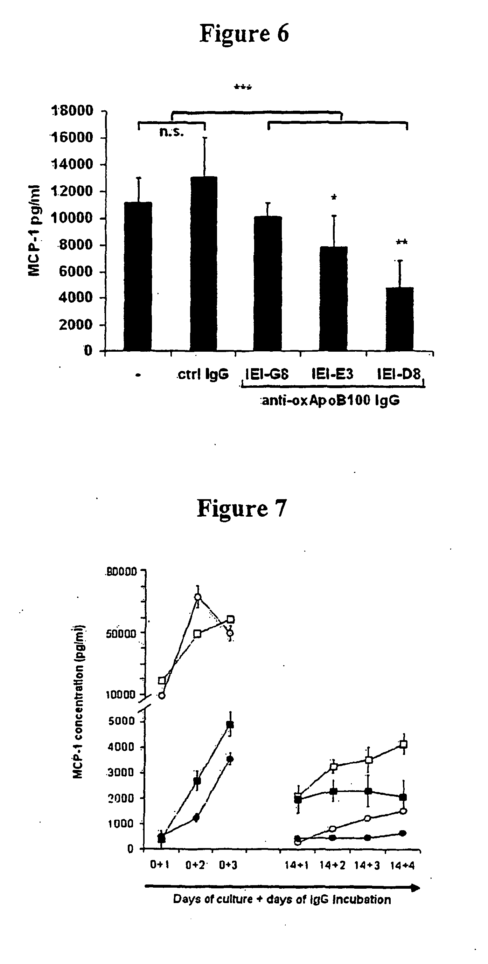

**Figure 6****.** Treatment of human monocyte derived macrophages with anti-oxidised ApoB-100 IgG blocks MCP-1 release. Human monocyte derived macrophages, pre-cultured for 10 days in the presence of human serum, were treated with 60µg/ml of anti-oxidised ApoB-100 IgG1 clones, negative control IgG, or saline only, as indicated, for 4 days. Supernatants were isolated and analysed for MCP-1 content using a commercially available ELISA. Data are means of triplicate values obtained from four donors with SEM.
**Figure 7****.** The MCP-1 blocking effect of anti-oxidised ApoB-100 IgG on atheroma-like macrophages is rapid and stable over time. Human monocytes or human monocyte derived macrophages were obtained from two donors (circles or squares, respectively). The cells were treated with 60µg/ml of the 2D03 antibody (closed symbols) or the control antibody (open symbols) either immediately (left set of graphs) or after pre-culture of the monocytes for 14 days in the presence of human serum (right set of graphs). Supernatants were isolated and analysed for MCP-1 CC chemokine content using a commercially available ELISA. Data are means of triplicate values with SEM.

### Example 1: Generation of human antibodies with high affinity and specificity for epitopes present on oxidised LDL

Human antibody fragments binding with specificity for oxidised LDL were selected from the n-CoDeR library essentially as described (Schiopu *et al,* 2004). Briefly; the library was expressed with phage display and selected for binding to a mixture of biotinylated oxidized peptides derived from ApoB-100. Binding phages were selected with avidin coated magnetic beads in 2-3 rounds of selection (Soderlind et al, (2000) Nature BioTechnol. 18: 852-856) and finally screened for binding to MDA- and Cu-oxidised forms of LDL and ApoB-100. The antibodies bound to MDA and Cu oxidised forms of LDL and ApoB-100 but not to their native, non-oxidised forms (Figure 1). The binding and affinity of the antibodies are exemplified in Figure 2 in which the binding of antibody 2D03 is compared with that of antibody IEI-E3. A comparison of the binding specificities of 2D03 and IEI-E3 found that they had similar, but not identical specificities for MDA-modified ApoB-100 derived peptides, and 2D03 gave a higher signal in the assay (Figure 3).

As shown in Figure 4, the antibodies also bound with specificity to human atherosclerotic plaques but not to normal tissue as assessed by immunohistochemistry.

### Example 2: Regression of atherosclerotic plaques in animal model using immunotherapy, based on passive immunisation

### Methods

### Antibodies

Selected scFv with desired properties were transferred to full length human IgG1 antibody format using previously described methods (Schiopu *et al,* 2004)

### Mice: passive immunisation and tissue preparation

Male LDLR^{-/-} ApoBec mice on C57BL/6 background from Jackson Laboratories (Bar Harbor, ME, USA) were used in the present study. From 4 weeks of age the mice were fed a high cholesterol diet (0.15% cholesterol, 21% fat, Lactamin AB, Kimstad, Sweden) provided *ad libitum.* One week before the first immunisation the diet was changed to normal chow. At 25 weeks of age one group of mice was sacrificed to serve as a control for the level of plaque formation at the initiation of the experiment and three groups of the remaining mice were injected intraperitoneally with 1 mg/dose (0.5 mL) of the human IgG1 antibodies directed to MDA modified ApoB-100 peptides or the control antibody respectively. Nonspecific human IgG1 antibodies directed to fluorescein isothiocyanate (FITC-8) were used as control. The injections were repeated 2 times at 1-week intervals.

The mice were humanely killed at 29 weeks of age by exsanguination through cardiac puncture under anesthesia with 300 µL of distilled water, fentanyl/fluanisone and midazolam (2:1:1, vol/vol/vol), administered intraperitoneally. After whole body perfusion with phosphate buffered saline (PBS) followed by Histochoice (Amresco, Solon, Ohio), the heart was dissected out and stored in Histochoice at 4°C until processing. The descending aorta was dissected free of external fat and connective tissue, cut longitudinally, and mounted *en-face* lumen side-up on ovalbumin- (Sigma, St. Louis, Missouri) coated slides (termed flat preparation) (Brånen *et al,* 2001). The local Animal Care and Use Committee approved the experimental protocol used in this study.

### Analysis of Plaque Area

Staining and quantification of plaque area in flat preparations of descending aorta was carried out done as previously described (Fredriksson *et al,* 2003). The plaque area was directly measured by microscopy and computer aided morphometry (Image Pro Plus) and the results expressed as average of plaque area/section.

### Statistical analysis

Data are presented as mean ± standard deviation. Analysis of the data was performed using two-tailed Mann-Whitney test. Statistical significance was considered at the level ≤ 0.05.

### Results

Recombinant human IgG1 antibodies directed against oxidised LDLwere compared in the study. We have previously shown that the IEI-E3 antibody effectively inhibits the early development of atherosclerosis in ApoE^{-/-} mice (Schiopu *et al,* 2004). A human IgG1 antibody directed against fluorescein. isothiocyanate, FITC-8, which does not bind native or oxidised LDL, was used as isotype control.

The effect of antibodies on atherosclerosis development was tested on LDLR^{-/-} ApoBec mice. The mice received 3 intraperitoneal injections of 1 mg antibody per dose at 25, 26 and 27 weeks of age and were sacrificed 2 weeks after the last injection, at 29 weeks of age. The general health status of the mice was not influenced by the antibody treatment. There were no significant differences among the test and control groups regarding weight and the plasma levels of cholesterol and triacylglycerol (data not shown). One group of animals were sacrificed at the onset of the experiment and served the purpose to determine a baseline level of plaque burden.

The extent of atherosclerosis was measured in Oil Red O stained *en-face* preparations of the thoracic and abdominal aorta. As shown in Figure 5, all test antibodies induced a significant regression of plaque burden in the treated animals as compared to the control antibody and to the baseline value obtained from mice at 25 weeks of age. The 2D03 antibody demonstrated the highest level of regression with a 51% effect (p=0.0003) compared to the FITC-8 antibody and with 60% (p=0.003) compared to the baseline value. The other antibodies also reduced plaque burden significantly (p<0.01) compared to either the FITC-8 group or the baseline value.

### Discussion

The present study showed that treatment with recombinant human IgG1 antibodies against epitopes on oxidised LDL significantly reduced the burden of atherosclerotic plaques in the descending aorta in an animal model. The more efficient binder 2D03 seemed to have a stronger effect than IEI-E3 or the other antibodies, but the differences between plaque area in the antibody treated groups were not significant. This finding demonstrates for the first time that antibodies binding to oxidised forms of LDL have the capability to induce regression of atherosclerotic plaques.

Antibodies found in plasma from human individuals and directed against oxidised LDL have been associated with disease extent, progression and degree of activity in many studies (Tsimikas *et al,* 2001, Salonen *et al,* 1992, Maggi *et al,* 1993). Levels of such antibodies may in the future become useful as markers for detecting the presence of vulnerable plaques in cardiovascular patients (Nilsson and Fredriksson, 2004, Nilsson and Kovanen, 2004).

Patients at high risk for myocardial infarction and stroke need immediate, fast acting and effective treatment. Statins (HMG-CoA inhibitors) are efficient in preventing acute cardiovascular events by reducing plasma cholesterol content and by additional mechanisms, yet to be clarified. Continuous efforts are made to develop new means of treatment, which, rather than replacing statins, would add to their efficacy by using different, complementary mechanisms.

A direct, immediately effective antibody therapy could therefore be the answer for the vulnerable patients on the verge of a life-threatening cerebrovascular or cardiac episode. We have shown in the present study that by transferring the mice from the western-type diet to normal chow followed by treatment with 3 doses of antibodies the extent of atherosclerosis decreased by 50% over a period of 4 weeks. As mentioned above, the mice already had complex atherosclerotic plaques at the time of initiation of the treatment.

The antibody treatment may be even more effective in humans than in mice. The antibodies that we used were human IgG1 antibodies generated against human oxLDL ApoB-100. The homology between human and mouse ApoB-100 is not perfect, impairing to a certain extent human antibody binding to mouse oxLDL. The immune system of the mice reacts to the foreign human protein generating mouse anti-human IgG1 antibodies that correlate negatively with the amount of injected antibodies still present in plasma at the time of euthanisation, as we have previously shown (Schipou *et al,* 2004). These antibodies might reduce the effectivenes of the human IgG1 treatment in mice by blocking their binding sites or by inducing their clearance from the circulation.

### Example 3: Modulation of macrophage inflammatory activity with antibodies against oxidised epitopes on LDL

Molecular mechanisms underlying the atheroprotective effects of anti-oxidised ApoB-100 (oxApoB-100) antibody treatment are incompletely characterised. However, the role of macrophages in plaque development and plaque homeostasis (Li and Glass, 2002), and the observation that anti-oxApoB-100 antibody treatment decreases plaque macrophage content *in vivo* (Schiopu *et al,* 2004), point to mechanisms that regulate macrophage recruitment and function. The MCP-1 pathway, constituted by MCP-1 and its receptor CCR2, is the most important chemotactic director of monocyte and macrophage infiltration and migration, and has been implicated in the atherogenic process at different levels (Charo and Taubman, 2004). Consequently, we examined the possibility that the atheroprotective effects of anti-oxApoB-100 IgG include interference with the MCP-1 signalling pathway.

Treatment of human monocyte derived macrophages with anti-oxApoB-100 IgG was found to decrease MCP-1 release by up to 60% compared to cells treated with control IgG (Figure 6). The inhibitory effect was titratable with maximum effects being observed at 30 to 60µg/ml. The MCP-1 blocking effect of anti-oxApoB-100 antibodies on mature macrophages was rapid and stable over time (Figure 7). MCP-1 concentrations of supernatants isolated 24, 48, 72 or 96 hours following incubation with anti-oxApoB-100 IgG remained constant at low levels. In contrast, treatment with control IgG allowed a steady increase in MCP-1 concentrations over time. The MCP-1 blocking effect was even more pronounced when antibodies were incubated with freshly isolated monocytes (Figure 7). Treatment of monocytes with anti-oxApoB-100 IgG for 48 hours resulted in a striking 60-fold decrease in MCP-1 concentrations compared to control IgG treated cells.

Anti-oxApoB-100 IgG treatment was also found to regulate MCP-1 synthesis at the transcriptional level. Freshly isolated monocytes were incubated with anti-oxApoB-100 IgG or control IgG for two days. Cells were harvested and mRNA was isolated and quantitated by real time PCR. Gene expression was related to 18S RNA. Monocyte cells incubated with anti-oxApoB-100 IgG for two days showed an 80% decrease in MCP-1 mRNA levels compared to cells treated with control IgG (data not shown).

The MCP-1 blocking effect of anti-oxApoB-100 IgG was not due to a general cytotoxic effect. Macrophages harvested following treatment with anti-oxApoB-100 IgG were fully viable as determined by trypan blue staining and subsequent analysis in a light microscope.

Our findings suggest that the atheroprotective effects of anti-oxApoB-100 antibodies include inhibitory effects on monocyte and macrophage release of MCP-1. MCP-1 is pivotal for monocyte and macrophage recruitment and migration in health and disease. Specifically, deletion of the monocyte/macrophage tropic CC chemokine MCP-1 (Gu *et al,* 1998) or its corresponding receptor CCR2 (Boring *et al,* 1998) protects mice from atherosclerosis *in vivo*. *In* vitro stimulation of monocytes with MCP-1 was shown to promote monocyte transendothelial migration and in the presence of oxLDL was shown to promote macrophage to foam cell transformation (Tangirala *et al,* 1997). Without wishing to be bound by theory we speculate that treatment with anti-oxApoB-100 antibodies may provide a selective inhibition of MCP-1 release thus minimizing potential side effects of inhibiting monocyte migration to inflammatory sites unrelated to the atherosclerotic plaque.

### Example 4: Induction of regression of atherosclerotic plaques in animal model using immunotherapy based on active immunisation

Immunisation with oxLDL (Palinski *et al,* 1995, *Ameli et al,* 1996, Freigang *et al,* 1998, Zhou *et al,* 2001, Nilsson *et al,* 1997) or with ApoB-100 peptides (Fredriksson *et al,* 2003) has previously been shown to be effective in mice, and studies for creating a vaccine against atherosclerosis are currently underway (Nilsson *et al,* 2004; Hansson 2002; Sherer & Shoenfeld 2002; Zhou & Hansson 2004).

In order to study the ability of vaccination against peptides derived from oxidised ApoB-100 to reduce the size of atherosclerotic plaques, LDLR-/- ApoBec mice are fed a high fat diet for 21 weeks starting at week 4. When the mice are 25 weeks old, one group of animals is sacrificed and the extent of atherosclerosis in their descending aortas is determined. This group serves as a baseline group, to which the extent of atherosclerosis in other groups can be compared. Test animals, vaccinated with peptide or PBS only, are taken off the high fat diet and given injections for several weeks, after which the animals are sacrificed and the level of atherosclerosis is determined.

In Example 2, above, we have demonstrated that the extent of plaque burden is stable over a 4 week period from week 25 to week 29 when the animals are taken off the high fat diet. The analysis revealed that baseline animals had 10% of their descending aorta area covered by plaque at the age of 25 weeks. Vaccinated animals are shown to have a reduced plaque burden compared to the control group and the baseline group. This demonstrates that immunisation against epitopes found on oxidised forms of LDL can lead to a reduction in the burden of pre-existing plaques and suggests that vaccination may be an efficient mode for decreasing plaque burden in patients.

### References

1. Ameli S, et al (1996) Effect of immunization with homologous LDL and oxidized LDL on early atherosclerosis in hypercholesterolemic rabbits. Arterioscler Thromb Vasc Biol. 1996 Aug;16(8):1074-9
2. Boring, L., et al (1998) Decreased lesion formation in CCR2-/- mice reveals a role for chemokines in the initiation of atherosclerosis. Nature 394, 894-7
3. Brånén, L., et al, A procedure for obtaining whole mount mouse aortas that allows atherosclerotic lesions to be quantified. Histochem J, 2001. 33: p. 227-229
4. Carr AC, McCall MR, Frei B. Oxidation of LDL by myeloperoxidase and reactive nitrogen species: reaction pathways and antioxidant protection. Arterioscler Thromb Vasc Biol. 2000 Jul;20(7):1716-23
5. Charo, IF and Taubman MB. (2004) Chemokines in the pathogenesis of vascular disease. Circ Res. 95(9):858-66
6. Esterbauer H, Schaur RJ, Zollner H..Chemistry and biochemistry of 4-hydroxynonenal, malonaldehyde and related aldehydes. Free Radic Biol Med. 1991;11(1):81-128
7. Fredrikson, G.N., et al, Inhibition of atherosclerosis in apoE-null mice by immunisation with ApoB-100 peptide sequences. Arterioscler Thromb Vasc Biol, 2003. 23: p. 879-884
8. Freigang S, et al (1998) Immunization of LDL receptor-deficient mice with homologous malondialdehyde-modified and native LDL reduces progression of atherosclerosis by mechanisms other than induction of high titers of antibodies to oxidative neoepitopes. Arterioscler Thromb Vasc Biol. 1998 Dec;18(12):1972-82
9. Friedman P, et al (2002) Correlation of antiphospholipid antibody recognition with the structure of synthetic oxidized phospholipids. Importance of Schiff base formation and aldol concentration. J Biol Chem. 2002 Mar 1;277(9):7010-20
10. George, J., et al, "Hyperimmunisation of apo-E-deficient mice with homologous malondialdehyde low-density lipoprotein suppresses early atherogenesis. Atherosclerosis, 1998. 138(1): p. 147-52
11. Gu, L., et al (1998) Absence of monocyte chemoattractant protein-1 reduces atherosclerosis in low density lipoprotein receptor-deficient mice. Mol Cell 2, 275-81
12. Hansson, G.K., Vaccination against atherosclerosis: science or fiction? Circulation, 2002. 106(13): p. 1599-601
13. Heery JM, et al. (1995) Oxidatively modified LDL contains phospholipids with platelet-activating factor-like activity and stimulates the growth of smooth muscle cells. J Clin Invest. 1995 Nov;96(5):2322-30
14. Li, A.C. and Glass, C.K. (2002) The macrophage foam cell as a target for therapeutic intervention. Nat Med 8, 1235-42
15. Leitinger N, et al (1999) Role of group II secretory phospholipase A2 in atherosclerosis: 2. Potential involvement of biologically active oxidized phospholipids. Arterioscler Thromb Vasc Biol. 1999 May;19(5):1291-8
16. Maggi, E., et al, Specificity of autoantibodies against oxidised LDL as an additional marker for atherosclerotic risk Coron Artery Dis, 1993. 4(12): p. 1119-22
17. McIntyre TM, Zimmerman GA, Prescott SM. Biologically active oxidized phospholipids. J Biol Chem. 1999 Sep 3;274(36):25189-92
18. Meziere C, (1997) In vivo T helper cell response to retro-inverso peptidomimetics. J Immunol. 1997 Oct 1;159(7):3230-7.
19. Nicoletti A, et al (1998). Immunoglobulin treatment reduces atherosclerosis in apo E knockout mice. J Clin Invest. 1998 Sep 1;102(5):910-8
20. Nilsson J, et al (1997) Immunization with homologous oxidized low density lipoprotein reduces neointimal formation after balloon injury in hypercholesterolemic rabbits. J Am Coll Cardiol. 1997 Dec;30(7):1886-91
21. Nilsson, J. and G.N. Fredrikson, Atherosclerosis. Autoimmunity, 2004. 37(4): p. 351-5
22. Nilsson, J. and P.T. Kovanen, Will autoantibodies help to determine severity and progression of atherosclerosis? Curr Opin Lipidol, 2004. 15(5): p. 499-503
23. Nilsson, J., G.K. Hansson, and P.K. Shah, Immunomodulation of Atherosclerosis. Implications for Vaccine Development. Arterioscler Thromb Vasc Biol, 2004
24. Nissen SE, et al (2003). Effect of recombinant ApoA-I Milano on coronary atherosclerosis in patients with acute coronary syndromes: a randomized controlled trial JAMA. 2003 Nov 5;290(17):2292-300
25. Palinski W, et al (1989) Low density lipoprotein undergoes oxidative modification in vivo. Proc Natl Acad Sci USA. 1989 Feb;86(4):1372-6
26. Palinski, W., E. Miller, and J.L. Witztum. Immunisation of low density lipoprotein (LDL) receptor-deficient rabbits with homologous malondialdehyde-modified LDL reduces atherogenesis. Proc Natl Acad Sci U S A, 1995. 92(3): p. 821-5
27. Salonen, J.T., et al, Autoantibody against oxidised LDL and progression of carotid atherosclerosis. Lancet, 1992. 339(8798): p. 883-7
28. Schiopu A, et al (2004) Recombinant human antibodies against aldehyde-modified apolipoprotein B-100 peptide sequences inhibit atherosclerosis. Circulation. 2004 Oct 5;110(14):2047-52
29. Shah PK, et al (1998). Effects of recombinant apolipoprotein A-I(Milano) on aortic atherosclerosis in apolipoprotein E-deficient mice. Circulation. 1998 Mar 3;97(8):780-5
30. Sherer, Y. and Y. Shoenfeld, Immunomodulation for treatment and prevention of atherosclerosis. Autoimmun Rev, 2002. 1(1-2): p. 21-7
31. Smiley PL, et al (1991) Oxidatively fragmented phosphatidylcholines activate human neutrophils through the receptor for platelet-activating factor. J Biol Chem. 1991 Jun 15;266(17):11104-10
32. Smith J, et al (1995). Decreased atherosclerosis in mice deficient in both macrophage colony-stimulating factor (op) and apolipoprotein E. Proc. Natl. Acad Sci. USA Vol. 92, pp. 8264-8268, August 1995
33. Tangirala RK, Murao K, Quehenberger O. Regulation of expression of the human monocyte chemotactic protein-1 receptor (hCCR2) by cytokines. J Biol Chem. 1997 Mar 21;272(12):8050-6
34. Tsimikas S, Shortal BP, Witztum JL, Palinski W. In vivo uptake of radiolabeled MDA2, an oxidation-specific monoclonal antibody, provides an accurate measure of atherosclerotic lesions rich in oxidized LDL and is highly sensitive to their regression. Arterioscler Thromb Vasc Biol. 2000 Mar;20(3):689-97
35. Tsimikas, S., W. Palinski, and J.L: Witztum, Circulating autoantibodies to oxidised LDL correlate with arterial accumulation and depletion of oxidised LDL in LDL receptor-deficient mice. Arterioscler Thromb Vasc Biol, 2001. 21 (1): p. 95-100
36. Watson AD, et al (1999). Structural identification of a novel pro-inflammatory epoxyisoprostane phospholipid in mildly oxidized low density lipoprotein. J Biol Chem. 1999 Aug 27;274(35):24787-98
37. Witztum JL, Berliner JA. Oxidized phospholipids and isoprostanes in atherosclerosis. Curr Opin Lipidol. 1998 Oct;9(5):441-8
38. Yang C, et al (2001) Selective oxidation in vitro by myeloperoxidase of the N-terminal amine in apolipoprotein B-100. J Lipid Res. 2001 Nov;42(11):1891-6
39. Zhou X, et al (2001) LDL immunization induces T-cell-dependent antibody formation and protection against atherosclerosis. Arterioscler Thromb Vasc Biol. 2001 Jan;21(1):108-14
40. Zhou, X. and G.K. Hansson, Immunomodulation and vaccination for atherosclerosis. Expert Opin Biol Ther, 2004. 4(4): p. 599-612.
41. Sharif F, et al (2004) "Current status of catheter- and stent-based gene therapy." Cardiovasc Res. 64(2): 208-16.
42. Svensson EC, et al. (1999) Efficient and stable transduction of cardiomyocytes after intramyocardial injection or intracoronary perfusion with recombinant adeno-associated virus vectors. Circulation. 99: 201-5.
43. Melo LG et al, (2004) Gene and cell-based therapies for heart disease. FASEB J. 18(6): 648-63.

## Claims

1. Use of at least one antibody that selectively binds to an oxidised epitope of ApoB100 or an oxidised lipid epitope of LDL in the preparation of a medicament for inducing regression of atherosclerotic plaques in a human individual who has a cardiovascular disease associated with atherosclerosis selected from myocardial infarction and stroke.

2. An antibody that selectively binds to an oxidised epitope of ApoB100 or an oxidized lipid epitope of LDL for use in inducing regression of atherosclerotic plaques in a human individual who has a cardiovascular disease associated with atherosclerosis selected from myocardial infarction and stroke.

3. Use according to Claim 1 or an antibody for use according to Claim 2 wherein the epitope of ApoB100 is selected from the group consisting of SEQ ID NO: 1 to 38; or is a fragment comprising at least 6 consecutive amino acid residues of a peptide from the group consisting of SEQ ID NO: 1 to 38.

4. Use according to Claim 1 or 3 or an antibody for use according to Claim 2 or 3 wherein the oxidized epitope of ApoB100 or LDL comprises an epitope of ApoB100 or LDL that has been oxidized by exposure to copper or by malone dealdehyde.

5. Use according to Claim 1 or an antibody for use according to Claim 2 wherein the antibody is a humanized antibody.

6. Use according to Claim 1 or 5 or an antibody for use according to Claim 2 or 5 wherein the antibody is an antibody fragment.

7. Use or antibody for use according to Claim 6 wherein the antibody fragment is a single chain antibody fragment.

8. A composition comprising at least one antibody that selectively binds to an oxidized epitope of ApoB100 or an oxidized lipid epitope of LDL for use in inducing regression of atherosclerotic plaques in a human individual who has a cardiovascular disease associated with atherosclerosis selected from myocardial infarction and stroke.

9. A composition for use according to Claim 8 wherein the oxidized epitope of LDL is as defined in any of Claims 1-4.

10. A composition for use according to Claim 8 wherein the antibody is as defined in any of Claims 1-4.

11. The use of at least one antibody molecule as defined in Claim 1 and a statin, in the preparation of a medicament for combating a cardiovascular disease associated with atherosclerosis in a human individual who has a cardiovascular disease associated with atherosclerosis selected from myocardial infarction and stroke by inducing the regression of atherosclerotic plaques.

12. A medicament comprising an antibody as defined in Claim 1 and a statin, for use in combating a cardiovascular disease associated with atherosclerosis in a human individual who has a cardiovascular disease associated with atherosclerosis selected from myocardial infarction and stroke by inducting the regression of atherosclerotic plaques.

13. An antibody as defined in Claim 1 for use in combating a cardiovascular disease associated with atherosclerosis in a human individual who has a cardiovascular disease associated with atherosclerosis selected from myocardial infarction and stroke by inducing regression of atherosclerotic plaques, where the antibody is for administration in conjunction with a statin.

14. A use according to any of Claims 1, 3-7 or an antibody for use according to any of Claims 2-7 wherein the antibody comprises
three light chain CDRs that have the sequence of SEQ ID NO: 42,43 and 44 and
three heavy chain CDRs that have the sequence of SEQ ID NO: 39,40 and 41, or
three light chain CDRs that have the sequence of SEQ ID NO: 48,49 and 50, and
three heavy chain CDRs that have the sequence of SEQ ID NO: 45, 46 and 47.

## Patentansprüche

1. Verwendung wenigstens eines Antikörpers, der selektiv ein oxidiertes Epitop von ApoB100 oder ein oxidiertes Lipidepitop von LDL bindet, bei der Herstellung eines Medikaments zum Induzieren einer Regression atherosklerotischer Plaques in einem humanen Individuum, das unter einer mit Atherosklerose assoziierten Herzkreislauferkrankung leidet, die aus Myokardinfarkt und Schlaganfall ausgewählt ist.

2. Antikörper, der selektiv ein oxidiertes Epitop von ApoB100 oder ein oxidiertes Lipidepitop von LDL bindet, für die Verwendung zum Induzieren einer Regression atherosklerotischer Plaques in einem humanen Individuum, das unter einer mit Atherosklerose assoziierten Herzkreislauferkrankung leidet, die aus Myokardinfarkt und Schlaganfall ausgewählt ist.

3. Verwendung gemäß Anspruch 1 oder Antikörper für die Verwendung gemäß Anspruch 2, wobei das Epitop von ApoB100 aus der aus SEQ ID NO: 1 bis 38 bestehenden Gruppe ausgewählt ist oder ein Fragment ist, dass wenigstens 6 aufeinanderfolgende Aminosäurereste eines Peptids aus der aus SEQ ID NO: 1 bis 38 bestehenden Gruppe umfasst.

4. Verwendung gemäß Anspruch 1 oder 3 oder Antikörper für die Verwendung gemäß Anspruch 2 oder 3, wobei das oxidierte Epitop of ApoB100 oder LDL ein Epitop von ApoB100 oder LDL umfasst, dass durch Exposition gegen Kupfer oder durch Malondialdehyd oxidiert wurde.

5. Verwendung gemäß Anspruch 1 oder Antikörper für die Verwendung gemäß Anspruch 2, wobei der Antikörper ein humanisierter Antikörper ist.

6. Verwendung gemäß Anspruch 1 oder 5 oder Antikörper für die Verwendung gemäß Anspruch 2 oder 5, wobei der Antikörper ein Antikörperfragment ist.

7. Verwendung oder Antikörper für die Verwendung gemäß Anspruch 6, wobei das Antikörperfragment ein Fragment eines einzelkettigen Antikörpers ist.

8. Zusammensetzung, die wenigstens einen Antikörper umfasst, der selektiv ein oxidiertes Epitop von ApoB100 oder ein oxidiertes Lipidepitop von LDL bindet, für die Verwendung zum Induzieren einer Regression atherosklerotischer Plaques in einem humanen Individuum, das unter einer mit Atherosklerose assoziierten Herzkreislauferkrankung leidet, die aus Myokardinfarkt und Schlaganfall ausgewählt ist.

9. Zusammensetzung für die Verwendung gemäß Anspruch 8, wobei das oxidierte Epitop von LDL so ist, wie es in irgendeinem der Ansprüche 1-4 definiert ist.

10. Zusammensetzung für die Verwendung gemäß Anspruch 8, wobei der Antikörper so ist, wie er in irgendeinem der Ansprüche 1-4 definiert ist.

11. Verwendung wenigstens eines Antikörpermoleküls, wie es im Anspruch 1 definiert ist, und eines Statins bei der Herstellung eines Medikaments zur Bekämpfung einer mit Atherosklerose assoziierten Herzkreislauferkrankung in einem humanen Individuum, das unter einer mit Atherosklerose assoziierten Herzkreislauferkrankung leidet, die aus Myokardinfarkt und Schlaganfall ausgewählt ist, durch das Induzieren der Regression atherosklerotischer Plaques.

12. Medikament, das einen Antikörper, wie er im Anspruch 1 definiert ist, und ein Statin umfasst, zur Verwendung bei der Bekämpfung einer mit Atherosklerose assoziierten Herzkreislauferkrankung in einem humanen Individuum, das unter einer mit Atherosklerose assoziierten Herzkreislauferkrankung leidet, die aus Myokardinfarkt und Schlaganfall ausgewählt ist, durch das Induzieren der Regression atherosklerotischer Plaques.

13. Antikörper, wie er im Anspruch 1 definiert ist, zur Verwendung bei der Bekämpfung einer mit Atherosklerose assoziierten Herzkreislauferkrankung in einem humanen Individuum, das unter einer mit Atherosklerose assoziierten Herzkreislauferkrankung leidet, die aus Myokardinfarkt und Schlaganfall ausgewählt ist, durch das Induzieren der Regression atherosklerotischer Plaques, wobei der Antikörper für die Verabreichung zusammen mit einem Statin bestimmt ist.

14. Verwendung gemäß irgendeinem der Ansprüche 1, 3-7 oder Antikörper für die Verwendung gemäß irgendeinem der Ansprüche 2-7, wobei der Antikörper umfasst
drei CDRs der leichten Kette, welche die Sequenz der SEQ ID NO: 42, 43 und 44 aufweisen, und
drei CDRs der schweren Kette, welche die Sequenz der SEQ ID NO: 39, 40 und 41 aufweisen, oder
drei CDRs der leichten Kette, welche die Sequenz der SEQ ID NO: 48, 49 und 50 aufweisen, und
drei CDRs der schweren Kette, welche die Sequenz der SEQ ID NO: 45, 46 und 47 aufweisen.

## Revendications

1. Utilisation d'au moins un anticorps qui se lie sélectivement à un épitope oxydé d'ApoB100 ou à un épitope de lipide oxydé de LDL, pour la préparation d'un médicament destiné à provoquer une régression des plaques athéroscléreuses chez un individu humain qui présente une maladie cardiovasculaire associée à l'athérosclérose choisie parmi l'infarctus du myocarde et une attaque.

2. Anticorps qui se lie sélectivement à un épitope oxydé d'ApoB100 ou à un épitope de lipide oxydé des LDL pour utilisation pour provoquer une régression des plaques athéroscléreuses chez un individu humain qui présente une maladie cardiovasculaire associée à l'athérosclérose choisie parmi l'infarctus du myocarde et une attaque.

3. Utilisation selon la revendication 1 ou anticorps pour utilisation selon la revendication 2, dans laquelle ou dans lequel l'épitope d'ApoB100 est choisi dans le groupe consistant en SEQ ID NO: 1 à 38 ; ou est un fragment comprenant au moins 6 résidus d'acide aminé consécutifs d'un peptide du groupe consistant en SEQ ID NO: 1 à 38.

4. Utilisation selon la revendication 1 ou 3 ou anticorps pour utilisation selon la revendication 2 ou 3, dans laquelle ou dans lequel l'épitope oxydé d'ApoB100 ou de LDL comprend un épitope d'ApoB100 ou de LDL qui a été oxydé par une exposition au cuivre ou par du malonedialdéhyde.

5. Utilisation selon la revendication 1 ou anticorps pour utilisation selon la revendication 2, dans laquelle ou dans lequel l'anticorps est un anticorps humanisé.

6. Utilisation selon la revendication 1 ou 5 ou anticorps pour utilisation selon la revendication 2 ou 5, dans laquelle ou dans lequel l'anticorps est un fragment d'anticorps.

7. Utilisation ou anticorps pour utilisation selon la revendication 6, dans laquelle ou dans lequel le fragment d'anticorps est un fragment d'anticorps simple chaîne.

8. Composition comprenant au moins un anticorps qui se lie sélectivement à un épitope oxydé d'ApoB100 ou à un épitope de lipide oxydé de LDL pour utilisation pour provoquer une régression des plaques athéroscléreuses chez un individu humain qui présente une maladie cardiovasculaire associée à l'athérosclérose choisie parmi l'infarctus du myocarde et une attaque.

9. Composition pour utilisation selon la revendication 8, dans laquelle l'épitope oxydé des LDL est tel que défini dans l'une quelconque des revendications 1 à 4.

10. Composition pour utilisation selon la revendication 8, dans laquelle l'anticorps est tel que défini dans l'une quelconque des revendications 1 à 4.

11. Utilisation d'au moins une molécule d'anticorps telle que définie dans la revendication 1 et d'une statine, pour la préparation d'un médicament permettant de combattre une maladie cardiovasculaire associée à l'athérosclérose chez un individu humain qui présente une maladie cardiovasculaire associée à l'athérosclérose choisie parmi l'infarctus du myocarde et une attaque en provoquant la régression des plaques athéroscléreuses.

12. Médicament comprenant un anticorps tel que défini dans la revendication 1 et une statine pour utilisation pour combattre une maladie cardiovasculaire associée à l'athérosclérose chez un individu humain qui présente une maladie cardiovasculaire associée à l'athérosclérose choisie parmi l'infarctus du myocarde et une attaque en provoquant la régression des plaques athéroscléreuses.

13. Anticorps tel que défini à la revendication 1 pour utilisation pour combattre une maladie cardiovasculaire associée à l'athérosclérose chez un individu humain qui présente une maladie cardiovasculaire associée à l'athérosclérose choisie parmi l'infarctus du myocarde et une attaque en provoquant la régression des plaques athéroscléreuses, dans lequel l'anticorps est pour une administration conjointement avec une statine.

14. Utilisation selon l'une quelconque des revendications 1, 3 à 7 ou anticorps pour utilisation selon l'une quelconque des revendications 2 à 7, dans laquelle ou dans lequel l'anticorps comprend
trois CDR de chaîne légère qui ont la séquence de SEQ ID NO: 42, 43 et 44 et
trois CDR de chaîne lourde qui ont la séquence de SEQ ID NO: 39, 40 et 41, ou
trois CDR de chaîne légère qui ont la séquence de SEQ ID NO: 48, 49 et 50, et
trois CDR de chaîne lourde qui ont la séquence de SEQ ID NO: 45, 46 et 47.
